# EUROPÄISCHE PATENTSCHRIFT

(11) **EP 1 752 135 B1**
(45) Veröffentlichungstag und Bekanntmachung des Hinweises auf die Patenterteilung: **12.10.2011**
(21) Anmeldenummer: 06118563.3
(22) Anmeldetag: 08.08.2006
(51) Int. Cl.: A61K 8/85, A61K 8/37, A61K 8/35, A61K 8/49, A61Q 17/04

(54) **Kosmetische Zubereitung mit UV-Lichtschutzfiltern**
Cosmetic preparation with UV-filters
Préparation cosmétique contenant des filtres UV

(30) Priorität: 09.08.2005 DE 102005037546
(43) Veröffentlichungstag der Anmeldung: 14.02.2007
(73) Patentinhaber: Beiersdorf Aktiengesellschaft, 20253 Hamburg (DE)
(72) Erfinder: Mundt, Claudia, 28207, Bremen (DE); Steinforth, Melanie, 22303 Hamburg (DE); Lerg, Heike, 22303, Hamburg (DE)

(56) Entgegenhaltungen:
- EP-A- 1 498 103
- EP-A- 1 634 581
- DATABASE WPI Week 200377 Derwent Publications Ltd., London, GB; AN 2003-820597 XP002410614 & JP 2003 238332 A (KOKYU ARUKORU KOGYO KK) 27. August 2003 (2003-08-27)
- 'Material Safety Data Sheet "Risocast DA-H"' KOKYU ALCOHOL KOGYO CO.

## Beschreibung

Die vorliegende Erfindung betrifft eine kosmetische Zubereitung enthaltend ein oder mehrere UV-Lichtschutzfilter sowie hydriertes Rizinusöl Dimerdilinoleat.

Der Trend weg von der vornehmen Blässe hin zur "gesunden, sportlich braunen Haut" ist seit Jahren ungebrochen. Um eine gebräunte Hautfarbe zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.

Kosmetische Sonnenschutzmittel sind hoch komplexe Substanzmischungen, deren häufig mangelnde Homogenität und Stabilität bei der Lagerung und Anwendung (UV-Stabilität) den Kosmetiker immer wieder vor Probleme stellt.

Zubereitungen welche die UV-Lichtschutzfilter 4-Methoxyzimtsäure(2-ethylhexyl)ester oder 4-(tert.-Butyl)-4'-methoxydibenzoylmethan enthalten haben das Problem, dass diese Lichtschutzfilter selbst nicht besonders UV stabil sind und sich unter dem Einfluss des Sonnenlichtes zersetzen. Dies führt nicht allein zu einer Abnahme der Wirksamkeit im Verlaufe der Anwendung. Darüber hinaus können die Zersetzungsprodukte (und deren Folgeprodukte) zu Verträglichkeitsproblemen bei der Anwendung auf der Haut führen.

Zubereitungen mit einem Gehalt an schwer löslichen UV-Filtern wie 2,4-Bis-{[4-(2-ethyl-hexyl-oxy)-2-hydroxy]-phenyl}-6-(4-methoxyphenyl)-1,3,5-triazin oder 4,4',4"-(1,3,5-Triazin-2,4,6-triyltriimino)-tris-benzoësäure-tris(2-ethylheoylester) haben häufig das Problem, dass diese Filter leicht aus der Zubereitung ausfallen - die Zubereitungen selbst also nicht besonders lager- und temperaturstabil sind. Bei der Anwendung auf der Haut neigen diese Filtersubstanzen zur kristallförmigen Abscheidungen, wodurch die Filterleistung an sich reduziert wird.

Kosmetische Sonnenschutzmittel werden besonders häufig im Badeurlaub angewendet, bei welchem sich die Konsumenten abwechselnd dem Sonnen- und dem Wasserbad aussetzen. Bei einer derartigen Anwendung ist es wünschenswert, dass das Sonnenschutzmittel besonders wasserfest ist, damit beim Baden die Schutzwirkung nicht verloren geht. Wasserfeste Sonnenschutzmittel sind dem Fachmann an sich bekannt, doch weisen diese in der Regel den Nachteil auf, dass die Zubereitungen besonders klebrig (insbesondere gegenüber Sand) sind. Auch sind nach dem Stand der Technik nicht alle UV-Lichtschutzfiltersubstanzen für "wasserfeste" Formulierungen geeignet. Insbesondere 4-Methooyzimtsäure(2-ethylheoyl)ester oder 4-(tert.-Butyl)-4'-methooydibenzoylmethan, 2,4-Bis-{[4-(2-ethyl-heoyloxy)-2-hydroxy]-phenyl}-6-(4-methoxyphenyl)-1,3,5-triazin und 4,4',4"-(1,3,5-Triazin-2,4,6-triyitriimino)-tris-benzoesäure-tris(2-ethylhexylester) werden relativ leicht von der Haut abgewaschen. Auf der anderen Seite weisen diese Lichtschutzfiltersubstanzen ein besonders interessantes und für die Schutzbelange wertvolles UV-Absorptionsspektrum auf, auf das der kosmetische Chemiker bei der Formulierung von Zubereitungen mit gleichmäßiger Filterleistung und hoher UV-A-Balance (nach DIN 67502) kaum verzichten kann.

Es war daher die Aufgabe der vorliegenden Erfindung, die Mängel des Standes der Technik zu beseitigen und kosmetische Zubereitungen mit UV-Lichtschutzfiltern herzustellen, die besonders photostabil und wasserfest sind. Ferner sollten die Zubereitungen lager- und temperaturstabil sein.

Es war die Aufgabe der vorliegenden Erfindung, Zubereitungen mit einem Gehalt an 4-Methooyzimtsäure(2-ethylheoyl)ester, und/oder 4-(tert.-Butyl)-4'-methooydibenzoylmethan zu entwickeln, die gegenüber herkömmlichen Zubereitungen eine erhöhte Photostabilität und Wasserfestigkeit aufweisen.

Außerdem war es die Aufgabe der vorliegenden Erfindung, Zubereitungen mit den UV-Lichtschutzfiltern 2,4-Bis-{[4-(2-ethyl-hexyloxy)-2-hydroxy]-phenyl}-6-(4-methoxyphenyl)-1,3,5-triazin und/oder 4,4',4"-(1,3,5-Triazin-2,4,6- triyltriimino)-tris-benzoësäure-tris(2-ethylhexyl-ester) zu entwickeln, die im Vergleich zu herkömmlichen Zubereitungen eine geringere Neigung zur Kristallisation der Filter in der Zubereitung und eine erhöhte Wasserfestigkeit aufweisen. In diesem Zusammenhang war es das Ziel der vorliegenden Erfindung, Zubereitungen mit einem deutlich höheren Gehalt an 2,4-Bis-{[4-(2-ethyl-hexyloxy)-2-hydroxy]-phenyl}-6-(4-methooyphenyl)-1,3,5-triazin und/oder 4,4',4"-(1,3,5-Triazin-2,4,6-triyitriimino)-tris-benzoesäure-tris(2-ethylhexylester) in gelöster Form zu entwickeln, um kosmetische Formulierungen mit einem besonders gleichmäßigen UV-Filterspektrum bei hoher Lichtschutzfilterleistung zu erhalten.

Überraschend gelöst werden die Aufgaben durch eine kosmetische Zubereitung enthaltend
a) ein oder mehrere UV-Lichtschutzfilter sowie
b) hydriertes Rizinusöl Dimerdilinoleat (CAS 646054-62-8), dadurch gekennzeichnet, daß das Gewichtsverhältnis der UV-Lichtschutzfilter a) zu hydriertem Rizinusöl Dimerdilinoleat b) von 15:1 bis 1:1 beträgt.

Dabei beträgt das Gewichtsverhältnis der UV-Lichtschutzfilter zu hydriertem Rizinusöl Dimerdilinoleat bevorzugt von 15:1 bis 5:1.

Die erfindungsgemäßen Zubereitungen führen bei der Anwendung nach dem Sonnenbad zu einer natürlichen bräunlichen Restbräune der Haut, während die Zubereitungen des Standes der Technik zu einer unnatürlichen, rötlichen Restbräune der Haut führen. Durch den Zusatz von hydriertem Rizinusöl Dimerdilinoleat (CAS 646054-62-8) in kosmetische Sonnenschutzmittel wird der Lichtschutzfaktor (LSF) überraschend erhöht.

Kosmetische Zubereitungen mit hydriertem Rizinusöl Dimerdilinoleat sind aus der EP 1 498 103 und der WO 01/54660 bekannt, doch konnten diese Schriften nicht den Weg zur vorliegenden Erfindung weisen, da in ihnen keine Sonnenschutzmittel beschrieben werden.

Überraschend gelöst werden die Aufgaben ferner durch eine kosmetische Zubereitung enthaltend
a) 4-Methooyzimtsäure(2-ethylheoyl)ester (CAS 5466-77-3) und/oder 4-(tert.-Butyl)-4'-methoxydibenzoylmethan (CAS 70356-09-1) in einer Gesamtkonzentration von 2 bis 15 Gewichts-%, bezogen auf das Gesamtgewicht der Zubereitung sowie
b) hydriertes Rizinusöl Dimerdilinoleat (CAS 646054-62-8).

Dabei ist es erfindungsgemäß bevorzugt, wenn 4-Methoxyzimtsäure(2-ethylhexyl)ester (CAS 5466-77-3, 2-Ethylhexyl-4-methoxycinnamat INCl: Octyl Methoxycinnamat, Handelsname:
Parsol MCX) in einer Konzentration von 1 bis 10 Gewichts-% und besonders bevorzugt in einer Konzentration von 3 bis 10 Gewichts-%, jeweils bezogen auf das Gesamtgewicht der Zubereitung, in dieser eingesetzt wird.

Auch ist es erfindungsgemäß bevorzugt, wenn 4-(tert.-Butyl)-4'-methoxydibenzoylmethan (CAS 70356-09-1, INCI: Butylmethoxydibenzoylmethan, Handelsname: Parsol 1789) in einer Konzentration von 0,1 bis 5 Gewichts-% und besonders bevorzugt in einer Konzentration von 2 bis 5 Gewichts-%, jeweils bezogen auf das Gesamtgewicht der Zubereitung, in dieser eingesetzt wird.

Erfindungsgemäß vorteilhaft beträgt das Gewichtsverhältnis der UV-Lichtschutzfilter zu hydriertem Rizinusöl Dimerdilinoleat in diesem Falle von 15:1 bis 1:1 .

In dieser erfindungsgemäß vorteilhaften Ausführungsform ist es erfindungsgemäß bevorzugt, wenn die erfindungsgemäße Zubereitung weitere UV-Lichtschutzfilter gewählt aus der Gruppe der UV-Lichtschutzfilter 2,4-Bis-{[4-(2-ethyl-hexyloxy)-2-hydroxy]-phenyl}-6-(4-methoxyphenyl)-1,3,5-triazin; Dioctylbutylamidotriazon; 2,4-bis-[5-1(dimethylpropyl)benzoxazol-2-yl-(4-phenyl)-imino]-6-(2-ethylhexyl)-imino-1,3,5-triazin; 4,4',4"-(1,3,5-Triazin-2,4,6-triyitriimino)-tris-benzoesäure-tris(2-ethylhexylester); 2,4,6-Tribiphenyl-4-yl-1,3,5-triazin; Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethyl butyl)-phenol); Ethylhexylsalicylat; Terephthalidendicamphersulfonsäure; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; Dioctylbutylamidotriazon; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; Titandioxid; Zinkoxid enthält.

Diese zusätzlichen UV-Lichtschutzfilter werden erfindungsgemäß vorteilhaft in einer Gesamtkonzentration von 1 bis 20 Gewichts-%, bezogen auf das Gesamtgewicht der Zubereitung, dieser zugesetzt.

Überraschend gelöst werden die Aufgaben nicht zuletzt durch eine kosmetische Zubereitung enthaltend
a) 2,4-Bis-{[4-(2-ethyl-hexyloxy)-2-hydroxy]-phenyl}-6-(4-methoxyphenyl)-1,3,5-triazin und/oder 4,4',4"-(1,3,5-Triazin-2,4,6-triyltriimino)-tris-benzoesäure-tris(2-ethylhexylester) in einer Gesamtkonzentration von 0,1 bis 10 Gewichts-%, bezogen auf das Gesamtgewicht der Zubereitung sowie
b) hydriertes Rizinusöl Dimerdilinoleat (CAS 646054-62-8).

Dabei ist es erfindungsgemäß bevorzugt, wenn 2,4-Bis-{[4-(2-ethyl-hexyloxy)-2-hydroxy]-phenyl}-6-(4-methoxyphenyl)-1,3,5-triazin ((INCI: Bis-Ethylhexyloxyphenol Methoxyphenyl Triazin, erhältlich unter dem Handelsnamen Tinosorb S) in einer Konzentration von 0,1 bis 10 Gewichts-% und besonders bevorzugt in einer Konzentration von 0,5 bis 5 Gewichts-%, jeweils bezogen auf das Gesamtgewicht der Zubereitung in dieser eingesetzt wird.

Auch ist es erfindungsgemäß bevorzugt, wenn 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCl: Octyl Triazone), welches von der BASF Aktiengesellschaft unter der Warenbezeichnung UVINUL^{®} T 150 vertrieben wird) in einer Konzentration von 0,1 bis 10 Gewichts-% und besonders bevorzugt in einer Konzentration von 0,5 bis 5 Gewichts-%, jeweils bezogen auf das Gesamtgewicht der Zubereitung in dieser eingesetzt wird.

Erfindungsgemäß vorteilhaft beträgt das Gewichtsverhältnis der UV-Lichtschutzfilter zu hydriertem Rizinusöl Dimerdilinoleat in diesem Falle von 15:1 bis 1:1.

In dieser erfindungsgemäß vorteilhaften Ausführungsform ist es erfindungsgemäß bevorzugt, wenn die erfindungsgemäße Zubereitung weitere UV-Lichtschutzfilter gewählt aus der Gruppe der UV-Lichtschutzfilter Dioctylbutylamidotriazon; 2,4-bis-[5-1(dimethylpropyl)benzoxazol-2-yl-(4-phenyl)-imino]-6-(2-ethylhexyl)-imino-1,3,5-triazin; 2,4,6-Tribiphenyl-4-yl-1,3,5-triazin; Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3- [1,3,3,3-tetramethyl-1-[(trimethylsilyl)oxy]disiloxanyl]propyl]-phenol; 3-(4-Methylbenzyliden)campher; 3-Benzylidencampher; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisopentylester; Ethylhexylsalicylat; Terephthalidendicamphersulfonsäure; 2-Hydroxy-4-methoxybenzophenon; 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan ― Copolymer; Titandioxid; Zinkoxid enthält.

Diese zusätzlichen UV-Lichtschutzfilter werden erfindungsgemäß vorteilhaft in einer Gesamtkonzentration von 0,1 bis 20 Gewichts-%, bezogen auf das Gesamtgewicht der Zubereitung, dieser zugesetzt.

Es ist vorteilhaft im Sinne der vorliegenden Erfindung, wenn die Zubereitung hydriertes Rizinusöl Dimerdilinoleat (CAS 646054-62-8) in einer Gesamtkonzentration 0,1 von 20 bis Gewichts-% und bevorzugt von 0,5 bis 5 Gewichts-%, jeweils bezogen auf das Gesamtgewicht der Zubereitung, enthält. Hydriertes Rizinusöl Dimerdilinoleat (CAS 646054-62-8, INCl Hydrogenated Castor Oil Dimer Dilinoleate) kann bei der Firma Kokyu Alcohol Kogyo unter dem Namen Risocast DA-H erworben werden. Es handelt sich bei dieser Verbindung um ein Polyester aus hydriertem Rizinusöl (CAS 8001-78-3) und der Dimersäure (CAS 68783-41-5). Der Polyester weist in einer erfindungsgemäß bevorzugten Ausführungsform eine Summenformel von ungefähr C₈₀₁H₁₅₀₂O₉₇ sowie ein Molekulargewicht von ungefähr 13000 auf.

Erfindungsgemäße kosmetische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe, einen Schaum oder auch ein Aerosol darstellen.

Dabei ist es erfindungsgemäß bevorzugt, wenn die kosmetische Zubereitung in Form einer Emulsion und besonders bevorzugt, wenn die Zubereitung in Form einer O/W-Emulsion vorliegt. Bei der Herstellung der Emulsionen oder Dispersionen können alle bekannten Emulgatoren und Emulgatorsysteme problemlos verwendet werden.

Es ist erfindungsgemäß vorteilhaft, wenn die erfindungsgemäße Zubereitung Tocopherylacetat enthält. Tocopherylacetat ist erfindungsgemäß vorteilhaft in einer Konzentration von 0,01 bis 5 Gewichts-% und bevorzugt in einer Konzentration von 0,1 bis 0,5 Gewichts-%, bezogen auf das Gesamtgewicht der Zubereitung, in der Emulsion enthalten.

Die erfindungsgemäße Zubereitung kann erfindungsgemäß vorteilhaft als Salbe, Creme oder Lotion (ggf. sprühbar) vorliegen.

Die erfindungsgemäße Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Emulsion, erfindungsgemäß.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner (z.B. Dihydroxyaceton), Repellentien sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die Ölphase der erfindungsgemäßen Zubereitung kann alle herkömmlichen Bestandteile von in der Kosmetik eingesetzten Öl-, Fett- und Wachskomponenten enthalten.

Die erfindungsgemäße Zubereitung kann erfindungsgemäß vorteilhaft kosmetische Wirk- und Pflegestoffe enthalten, beispielsweise die nach der Kosmetikverordnung zugelassenen Konservierungsmittel oder Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin A, Vitamin A-Derivate, Vitamin C, Vitamin C-Derivate, Coenzym Q10, Kreatin, Taurin, Licochalkon und/oder alpha-Glycosylrutin in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Die erfindungsgemäße Zubereitung kann beliebige Mischungen von Duft- und Parfümstoffen in den unterschiedlichsten Konzentrationen enthalten.

### Vergleichsversuche

In einer in vivo Untersuchung wurde ein Sonnenschutzmittel mit/ohne hydriertem Rizinusöl Dimerdilinoleat (CAS 646054-62-8) auf seine Wasserfestigkeit und seinen Lichtschutzfaktor hin vermessen

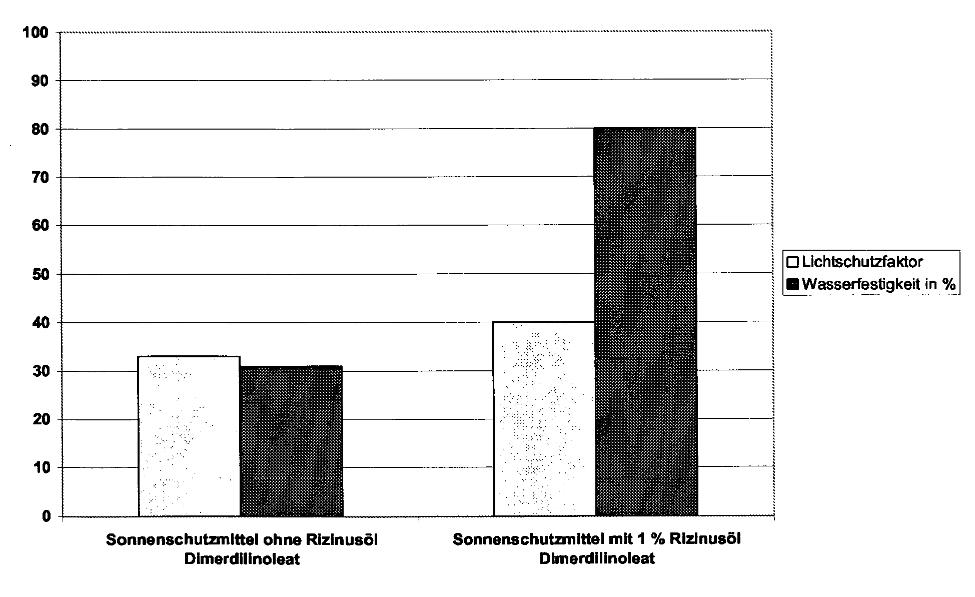

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

### Beispiele

In den folgenden Beispielen bedeutet:
• UVASorb® K2A = 2,4-bis-[5-1(dimethylpropyl)benzoxazol-2-yl-(4-phenyl)-imino]-6-(2- ethylhexyl)-imino-1,3,5-triazin [CAS Nr. 288254-16-0]
• Uvinul® A Plus = 2-(4'-Diethylamino-2'-hydoxybenzoyl)-benzoesäurehexylester (auch: Aminobenzophenon)

**1. O/W Emulsionen**

| | **1** | **2** | **3** | **4** | **5** | **6** | **7** |
|---|---|---|---|---|---|---|---|
| PEG-40 Castor Öl, Natrium Cetearyl Sulfat, Cetearyl Alkohol | | | | | | | 2,50 |
| Glycerinmonostearat (SE) | 1,00 | 2,00 | 3,00 | 1,00 | | 1,50 | 1,00 |
| Glyceryl Stearat Citrat | | | | | 2,00 | | |
| Stearinsäure | 3,00 | | 2,50 | 2,00 | | | |
| PEG-40 Stearat | | 2,00 | | | | 2,00 | |
| PEG-100 Stearat | | | 0,75 | | | | |
| Lauryl Methicon Copolyol | | | | 0,75 | | 0,50 | |
| Sorbitan Stearat | | | 0,75 | | | | |
| Cetyl Phosphat | | | 0,75 | | | | |
| Stearyl Alkohol | | | 3,00 | | 2,00 | 2,00 | |
| Cetyl Alkohol | 1,00 | 2,00 | | | 0,50 | | 1,50 |
| UVASorb® K2A | | | | 4,00 | | 5,00 | |
| Uvinul®A Plus | 2,50 | | | 0,25 | 1,00 | 0,50 | |
| Butylmethoxydibenzoylmethan | | | | | | | 4,00 |
| 4-Methylbenzylidencampher | | | 3,00 | | | | |
| Bis-Ethylhexyloxyphenol Methoxyphenyl Triazin | 1,00 | | | | | 1,00 | 3,00 |
| Dinatrium Phenyl Dibenzimidazol Tetrasulfonat | | | | 1,00 | | 2,00 | |
| Phenylbenzmidazol Sulfonsäure | | | | | | | 2,00 |
| Ethylhexyl Triazon | | | | 2,00 | | 2,00 | |
| Diethylhexyl Butamido Triazon | 2,00 | | | | | | |
| Ethylhexyl Methoxycinnamat | 3,50 | | | 10,00 | | | 9,00 |
| Octocrylen | | | | 5,00 | 9,00 | 7,50 | |
| Methylen Bis-Benztriazolyl Tetramethylbutylphenol | | | 2,00 | | 3,00 | | |
| Ethylhexylsalicylat | | 0,50 | 3,00 | | | | |
| Drometrizol Trisiloxan | | | 0,50 | | | 1,00 | |
| Terephtalidene Dicampher Sulfonsäure | | | | | | 2,00 | |
| Dimethylcodiethylbenzalmalonat | | | | | | 3,00 | |
| Titandioxid T 805 | 2,00 | | | | 1,00 | 0,50 | |
| Titandioxid MT-100Z | | | | 3,00 | 1,00 | | |
| C₁₂₋₁₅ Alkyl Benzoat | 1,00 | | | | | 2,00 | 5,50 |
| Octyldodecanol | 1,50 | | | 0,50 | | 4,00 | 5,50 |
| hydriertes Rizinusöl Dimerdilinoleat (CAS 646054-62-8) | 2,00 | 0,50 | 3,50 | 4,00 | 7,50 | 1,50 | 1,00 |
| Dicaprylylether | | | 3,50 | | 2,00 | | |
| Paraffinöl | | 6,00 | | | | | |
| Butylenglycol Dicaprylat/ Dicaprat | | | | 5,00 | | | |
| Cetearyl Isononanoat | 4,00 | | | | | 2,00 | |
| Dimethicon | 0,50 | 3,00 | 1,00 | | 2,00 | | |
| Cyclomethicon | | 3,00 | | 4,50 | | | |
| Dimethicon / Vinyl Dimethicon Crosspolymer | 4,00 | | | | | | |
| PVP Hexadecen Copolymer | | | | 0,50 | 1,00 | | |
| Glycerin | 7,50 | 10,00 | | 7,50 | 5,00 | | 1,00 |
| Xanthangummi | | 0,20 | 0,05 | | | | |
| Butylenglycol | 5,00 | | | | | 7,00 | |
| Ascorbinsäure | | | | | | 3,50 | |
| Tocopherol | 0,20 | | | | | | |
| Taurin | 1,00 | | | | | | |
| Vitamin E Acetat | | 0,40 | 0,25 | 0,50 | 0,75 | | 0,10 |
| Retinol | | | | | 0,05 | | |
| Natrium Stärke Octenylsuccinat | | | | 0,20 | | 0,25 | 1,50 |
| Maisstärke | | 0,5 | | | | | 3,00 |
| Fucogel® 1000 | | | 1,50 | | | 5,00 | |
| Dihydroxyaceton | | 5,00 | | | | | |
| DMDM Hydantoin | 0,60 | | 0,40 | 0,20 | | | |
| Methylparaben | | 0,10 | 0,25 | | 0,50 | | 0,10 |
| Phenoxyethanol | 0,40 | 0,50 | | 0,40 | 0,50 | | 0,60 |
| EDTA | 0,20 | | 0,35 | 0,50 | 0,02 | | 0,20 |
| Ethanol | 2,00 | | 1,50 | | 3,00 | 5,00 | 8,00 |
| Insekt Repellent 3535 | | | 5,00 | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (Natriumhydroxid, Kaliumhydroxid) | qs | qs | qs | qs | qs | qs | qs |
| (Natriumhydroxid, Kaliumhydroxid) | | | | | | | |
| pH-Wert | 6,0-7,5 | 4,5-7,0 | 6,5-8,5 | 5,0-7,0 | 6,0-8,0 | 4,0-6,0 | 5,0-7,5 |

**O/W Emulsionen**

| | **8** | **9** | **10** | **11** | **12** |
|---|---|---|---|---|---|
| Glycerinmonostearat (SE) | | | | 1,00 | |
| Glyceryl Stearat Citrat | 2,00 | 2,00 | 1,50 | | |
| Polyglyceryl-3- Methylglucose Distearat | | | | | 4,50 |
| Stearyl Alkohol | 2,00 | 2,00 | | | |
| Cetyl Alkohol | | | 2,00 | | 4,50 |
| Bis-Ethylhexyloxyphenol Methoxyphenyl Triazin | 2,00 | 2,00 | 1,50 | | |
| Diethylhexyl Butamido Triazon | | 1,00 | 2,00 | | |
| Ethylhexyl Methoxycinnamat | 2,00 | 6,00 | 5,00 | | |
| Octocrylen | 2,00 | 9,00 | | | |
| Titandioxid T 805 | | 3,00 | 2,00 | | |
| C12-15 Alkyl Benzoat | | | 3,00 | 3,00 | 1,00 |
| hydriertes Rizinusöl Dimerdilinoleat (CAS 646054-62-8) | 1,00 | 4,00 | 6,00 | 2,00 | 3,00 |
| Hydriertes Kokosfettsäureglycerid | 1,00 | 1,00 | 3,00 | | |
| Dicaprylylether | 5,00 | 2,00 | 6,00 | | |
| Octyldodecanol | 6,00 | 5,00 | 4,00 | 1,00 | 2,00 |
| Butylenglycol Dicaprylat/ Dicaprat | 2,00 | 2,00 | | | |
| Caprylsäure/Caprinsäuretriglyc erid | | | | 2,00 | 1,50 |
| Dimethicon | | | 2,00 | | |
| Cyclomethicon | 2,00 | 1,00 | | 3,00 | |
| Sorbitol | | | | 2,50 | |
| Acrylat/C10-30 Alkyl Acrylat Crosspolymer | 0,10 | 0,10 | 0,05 | | |
| PVP Hexadecen Copolymer | 0,50 | 0,50 | | | |
| Glycerin | 8,00 | 6,00 | 5,00 | 3,00 | 3,00 |
| Xanthangummi | 0,40 | 0,40 | 0,25 | 0,30 | 0,10 |
| Butylenglycol | | | 3,00 | 3,00 | |
| Linolsäure | 1,00 | | | | |
| Nachtkerzenöl | | 1,00 | | | |
| Genistein | 0,05 | | | | |
| Silymarin | | 0,1 | | | |
| Phosphatidylcholin | | | | 0,06 | |
| Silybin | | | | 0,03 | |
| Carnitin | | | 0,5 | | |
| Vitamin E Acetat | 0,50 | | 0,30 | 0,40 | 0,40 |
| Dihydroxyaceton | | | | 5,00 | 4,00 |
| DMDM Hydantoin | 0,60 | 0,60 | 0,50 | | |
| Methylparaben | | | | 0,30 | 0,30 |
| Phenoxyethanol | 0,40 | 0,40 | 0,35 | 0,50 | 0,50 |
| EDTA | 1,00 | 1,00 | 1,00 | | 1,00 |
| Ethanol | | | 3,00 | 3,00 | |
| Insekt Repellent 3535 | | | | 4,00 | 5,00 |
| Parfüm | 0,40 | 0,40 | 0,40 | 0,40 | 0,40 |
| Wasser | ad 100 | ad 100 | ad 100 | ad 100 | ad 100 |
| Neutralisationsmittel (Natriumhydroxid, Kaliumhydroxid) | qs | qs | qs | qs | qs |
| pH-Wert | 5,0-7,0 | 5,0-7,0 | 5,0-7,0 | 5,0-7,0 | 5,0-7,0 |

**O/W-Emulsionen**

| | **13** | **14** | **15** | **16** | **17** | **18** | **19** |
|---|---|---|---|---|---|---|---|
| Glyceryl Stearat, Ceteareth-12, Ceteareth-20, Cetearyl Alcohol, Cetyl Palmitat | | | | | | | 1,50 |
| Glycerinmonostearat (SE) | | 4,00 | | | | | |
| Glyceryl Stearat Citrat | 2,00 | | | | | | |
| Polyglyceryl-3- Methylglucose Distearat | | | | | 2,00 | | |
| Cetearyl Glucosid & Cetearyl Alkohol | | | | | | 2,00 | |
| Triceteareth-4 Phosphat | | | 1,20 | | | | |
| Trilaureth-4 Phosphat | | | | 2,00 | | | |
| Ceteareth-6 | | | 0,50 | 0,50 | | | |
| Sorbitan Stearat | | | 0,75 | 1,00 | 1,00 | | |
| Cetyl Phosphat | | | | | 2,00 | | |
| Stearyl Alkohol | | 2,50 | 3,00 | | | | 1,50 |
| Cetyl Alkohol | 2,50 | 1,00 | | | 0,50 | 2,00 | 2,00 |
| Phenethyl Benzoat | 1,50 | | | 0,50 | | 4,00 | 10,00 |
| hydriertes Rizinusöl Dimerdilinoleat (CAS 646054-62-8) | 2,00 | 1,50 | 3,50 | 4,00 | 0,50 | 2,50 | 1,00 |
| UVASorb® K2A | 1,00 | | | 4,00 | | 5,00 | |
| Uvinul® A Plus | 3,00 | 2,50 | 0,50 | 0,25 | 1,00 | 0,50 | |
| Butylmethoxydibenoylmethan | | | | | | | 4,50 |
| Bis-Ethylhexyloxyphenol Methoxyphenyl Triazin | | 2,00 | | | | 1,00 | 0,50 |
| Dinatrium Phenyl Dibenzimidazol Tetrasulfonat | | | | 1,00 | | 2,00 | |
| Phenylbenzmidazol Sulfonsäure | | | | | 3,00 | | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 9,00 | 7,50 | 2,50 |
| Methylen Bis-Benztriazolyl Tetramethylbutylphenol | | | 2,00 | | 3,00 | | |
| Ethylhexylsalicylat | | | | | | | 5,00 |
| Drometrizol Trisiloxan | | | 0,50 | | | 1,00 | |
| Terephtalidene Dicampher Sulfonsäure | | 3,00 | | | | | |
| Dimethylcodiethylbenzalmalonat | | | 5,00 | | | | |
| Titandioxid MT-100Z | 1,00 | | | 3,00 | 1,00 | | |
| Zinkoxid Z-Cote HP1 | | | | | | 3,00 | |
| Corapan TQ® | | | | | | | 6,00 |
| C₁₂₋₁₅ Alkyl Benzoat | | 2,50 | | | | 7,00 | 5,00 |
| Dicaprylylether | | | 3,50 | | 2,00 | | |
| Butylenglykol Dicaprylat/ Dicaprat | 5,00 | | | 5,00 | 3,00 | | |
| Cetearyl Isononanoat | | 4,00 | | | | 2,00 | 2,00 |
| Dimethicon | | 0,50 | 1,00 | | 2,00 | | |
| Cyclomethicon | 2,00 | | | 4,50 | | | 0,50 |
| Dimethicon / Vinyl Dimethicon Crosspolymer | | 4,00 | | | | | 0,50 |
| PVP Hexadecen Copolymer | 0,50 | | | 0,50 | 1,00 | | 1,00 |
| Glycerin | 3,00 | 7,50 | | 7,50 | 5,00 | | 20,00 |
| Xanthangummi | 0,15 | | 0,05 | | | | 0,30 |
| Butylenglycol | 7,00 | 5,00 | | | | 7,00 | |
| Süßholzextrakt | | | | | | 0,5 | |
| Licochalcon | | | 0,05 | | | | |
| Curcumin | 0,05 | | | | | | |
| beta-Carotin | | | | 0,1 | | | |
| Ceramid III | | | | | 0,3 | | |
| Isoserinol | | 1,0 | | | | | |
| Vitamin E Acetat | 0,50 | | 0,25 | 0,50 | 0,75 | | 1,00 |
| Dioic Acid | 0,25 | | | 0,20 | | 0,25 | |
| Fucogel® 1000 | | | 1,50 | | | 5,00 | |
| DMDM Hydantoin | | 0,60 | 0,40 | 0,20 | | | |
| Methylparaben | 0,15 | | 0,25 | | 0,50 | | |
| Phenoxyethanol | 1,00 | 0,40 | | 0,40 | 0,50 | | 0,60 |
| EDTA | | 0,20 | 0,35 | 0,50 | 0,02 | | 0,03 |
| Alkohol | | 2,00 | 1,50 | | 3,00 | 5,00 | 1,00 |
| Insekt Repellent 3535 | | | 5,00 | | | | |
| Meersalz | 0,1 | | | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (NaOH, KOH) | qs | qs | qs | qs | qs | qs | qs |
| pH-Wert | 4,5-6,0 | 4,5-7,0 | 5,5-7,5 | 5,0-7,0 | 5,5-7,5 | 4,0-7,0 | 4,0-7,5 |

**1. O/W Emulsionen**

| | **20** | **21** | **22** | **23** | **24** | **25** | **26** |
|---|---|---|---|---|---|---|---|
| PEG-40 Castor Öl, Natrium Cetearyl Sulfat, Cetearyl Alkohol | 2,50 | 3,50 | 2,50 | 2,25 | 2,00 | 3,00 | 2,50 |
| Glycerinmonostearat (SE) | 1,00 | 1,50 | 1,00 | 1,25 | 1,50 | 1,00 | 1,00 |
| Cetyl Alkohol | 1,50 | 1,00 | 1,50 | 2,00 | 1,00 | 1,00 | 1,50 |
| Uvinul® A Plus | | | | | 4,00 | 2,00 | |
| Butylmethoxydibenzoylmethan | 4,90 | 4,50 | 2,00 | 3,50 | 2,50 | 3,50 | 4,50 |
| 4-Methylbenzylidencampher | | | | | | | |
| Bis-Ethylhexyloxyphenol Methoxyphenyl Triazin | 1,50 | 3,00 | 2,00 | 2,00 | | 1,00 | 3,00 |
| Dinatrium Phenyl Dibenzimidazol Tetrasulfonat | | | 1,00 | | | | |
| Phenylbenzmidazol Sulfonsäure | | 2,00 | 2,00 | | | 2,50 | 2,00 |
| Ethylhexyl Triazon | | 1,50 | | 3,00 | | | |
| Diethylhexyl Butamido Triazon | 1,00 | | 0,50 | 1,00 | 1,50 | | |
| Ethylhexyl Methoxycinnamat | 4,50 | | 3,00 | 4,50 | | | 9,50 |
| Octocrylen | 3,00 | | | 1,00 | | | |
| Ethylhexylsalicylat | | | | 4,50 | 3,50 | 2,00 | |
| Polysilicon 15 | | | | | | 2,00 | |
| Titandioxid T 805 | | | | | | | 1,00 |
| C₁₂₋₁₅ Alkyl Benzoat | 5,50 | 5,50 | 3,50 | 5,50 | 4,50 | 5,50 | 5,50 |
| Octyldodecanol | 5,50 | 5,50 | 7,50 | 5,50 | 4,50 | 5,50 | 5,50 |
| hydriertes Rizinusöl Dimerdilinoleat (CAS 646054-62-8) | 2,00 | 0,50 | 1,00 | 1,50 | 3,00 | 6,00 | 1,75 |
| Dicaprylylether | | | | | | 1,00 | |
| Paraffinöl | | 2,00 | | | | | |
| Butylenglycol Dicaprylat/ Dicaprat | 1,00 | | | | | | |
| Cetearyl Isononanoat | | | | | 2,00 | | |
| Dimethicon | | | 2,00 | | | | |
| Cyclomethicon | | | 5,00 | 3,00 | | | |
| Dimethicon / Vinyl Dimethicon Crosspolymer | | | | | 0,30 | | |
| PVP Hexadecen Copolymer | | | | | | 0,50 | |
| Glycerin | 7,50 | 1,00 | 2,00 | 1,00 | 1,00 | 1,00 | 1,00 |
| Methylpropandiol | | | 2,00 | | 1,00 | | |
| Butylenglycol | | | | | | 2,00 | |
| Vitamin E Acetat | 2,00 | 0,10 | 0,50 | 0,10 | | 0,75 | |
| Retinol | | | | | | | |
| Natrium Stärke Octenylsuccinat | 1,50 | 1,50 | 1,50 | | 1,50 | 1,00 | 1,50 |
| Maisstärke | 2,00 | 3,00 | | 1,00 | 3,00 | 4,00 | 3,00 |
| Dihydroxyaceton | | | 2,00 | | | | |
| Methylparaben | 0,10 | 0,10 | 0,20 | | 0,10 | | 0,10 |
| Phenoxyethanol | 0,60 | 0,50 | 0,60 | 0,60 | 0,60 | | 0,60 |
| EDTA | 0,20 | 0,20 | 0,20 | 0,20 | 0,20 | 0,20 | 0,20 |
| Ethanol | 8,00 | 8,00 | 8,00 | 8,00 | 8,00 | 8,00 | 8,00 |
| Insekt Repellent 3535 | | | | 0,50 | | | |
| Parfüm | 0,40 | 0,40 | 0,40 | 0,40 | 0,40 | 0,40 | 0,40 |
| Wasser | ad 100 | ad 100 | ad 100 | ad 100 | ad 100 | ad 100 | ad 100 |
| Neutralisationsmittel (Natriumhydroxid, Kaliumhydroxid) | qs | qs | qs | qs | qs | qs | qs |
| pH-Wert | 5,0-7,5 | 5,0-7,5 | 5,0-7,5 | 5,0-7,5 | 5,0-7,5 | 5,0-7,5 | 5,0-7,5 |

**2. Schaumförmige O/W- Emulsionen:**

| | | **1** | | **2** |
|---|---|---|---|---|
| | Gew.-% | Vol.-% | Gew.-% | Vol.-% |
| Stearinsäure | 5,00 | | 1,00 | |
| Cetylalkohol | 5,50 | | | |
| Cetylstearylalkohol | | | 2,00 | |
| PEG-40 Stearat | 8,50 | | | |
| PEG-20-Stearat | | | 1,00 | |
| Caprylsäure/Caprinsäuretriglycerid | 4,00 | | 2,00 | |
| C12-15 Alkyl Benzoat | 7,00 | | 8,50 | |
| hydriertes Rizinusöl Dimerdilinoleat (CAS 646054-62-8) | 0,50 | | 2,50 | |
| Cyclomethicon | 4,00 | | | |
| Dimethicon | | | 0,50 | |
| Octyl isostearat | | | 5,00 | |
| Myristyl Myristat | | | 2,00 | |
| Ceresin | 1,50 | | | |
| Glycerin | 5,00 | | 10,00 | |
| UVASorb® K2A | 2,00 | | | |
| Uvinul A Plus® | 2,00 | | 1,50 | |
| Terephthaliden Dicamphersulfonsäure | 0,50 | | | |
| Drometrizol Trisiloxan | 1,50 | | | |
| Ethylhexylmethoxycinnamat | 5,00 | | 4,00 | |
| Ethylhexyltriazon | | | 3,00 | |
| Octocrylene | 5,00 | | | |
| Titandioxid Uvinul T 805 | 1,00 | | | |
| BHT | | | 0,02 | |
| Na₂H₂EDTA | 0,50 | | 0,10 | |
| Parfüm, Konservierungsmittel, | q.s. | | q.s. | |
| Farbstoffe, usw. | q.s. | | q.s. | |
| Kaliumhydroxid | q.s. | | q.s. | |
| Wasser | ad 100,00 | | ad 100,00 | |
| | pH-Wert eingestellt auf 6,5-7,5 | | pH-Wert eingestellt auf 5,0-6,0 | |
| Emulsion 1 | | 70 | | |
| Emulsion 2 | | | | 35 |
| Gas (Stickstoff, Sauerstoff oder Kohlendioxid) | | 30 | | |
| Gas (Luft) | | | | 65 |

**weitere Schaumförmige O/W- Emulsionen:**

| | **3** | **4** | **5** |
|---|---|---|---|
| Stearinsäure | 2,00 | 2,00 | |
| Palmitinsäure | | | 1,50 |
| Cetylalkohol | 2,50 | 2,00 | |
| Stearylalkohol | | | 3,00 |
| PEG-100 Stearat | | | 3,50 |
| PEG-40 Stearat | | 2,00 | |
| PEG-20-Stearat | 3,00 | | |
| Sorbitanstearat | | 0,80 | |
| C12-15 Alkyl Benzoat | 5,00 | | |
| C12-13 Alkyl Tartrat | | | 4,00 |
| hydriertes Rizinusöl Dimerdilinoleat (CAS 646054-62-8) | 2,00 | 1,00 | 0,50 |
| Butylenglycol Dicaprylat/Dicaprat | | 6,00 | |
| Dicaprylyl Ether | | | 2,00 |
| Cyclomethicon | | 2,00 | 3,00 |
| Butylenglycol | 1,00 | | |
| Isohexadecan | 2,00 | | |
| Propylenglykol | | | 5,00 |
| Glycerin | 5,00 | 7,00 | |
| UVASorb® K2A | | | 2,00 |
| Uvinul A Plus® | 2,00 | | |
| Ethylhexyltriazon | 2,00 | 2,00 | 2,00 |
| Octocrylen | 2,00 | | |
| Bis-Ethylhexyloxyphenol Methoxyphenyltriazin | | 3,00 | 3,00 |
| Vitamin E Acetat | | 0,5 | |
| BHT | | | 0,10 |
| Na₂H₂EDTA | 0,50 | | |
| Parfüm, Konservierungsmittel | q.s. | q.s. | q.s. |
| Farbstoffe, usw. | q.s. | q.s. | q.s. |
| Natriumhydroxid | q.s. | | q.s. |
| Kaliumhydroxid | | q.s. | |
| Wasser | ad 100,0 | ad 100,0 | ad 100,0 |

**weitere Schaumförmige O/W- Emulsionen:**

| | **6** | **7** | **8** | **9** |
|---|---|---|---|---|
| Stearinsäure | 1,50 | | | |
| Palmitinsäure | | | 3,00 | 3,00 |
| Cetylalkohol | | 3,00 | | |
| Cetylstearylalkohol | | | 2,00 | 2,00 |
| Stearylalkohol | 3,00 | | | |
| PEG-100 Stearat | | 4,00 | | |
| PEG-40 Stearat | 3,00 | | | |
| PEG-20-Stearat | | | 3,00 | 3,00 |
| Sorbitanstearat | 1,00 | | | |
| Tridecyl Trimellitate | | 5,00 | | |
| C12-15 Alkyl Benzoat | | | 3,00 | 3,00 |
| hydriertes Rizinusöl Dimerdilinoleat (CAS 646054-62-8) | 2,00 | 1,00 | 0,10 | 6,00 |
| Butylenglycol Dicaprylat/Dicaprat | 8,00 | | | |
| Octyldodecanol | | 2,00 | | |
| Kokosfettsäureglycerid | | | | 2,00 |
| Dicaprylyl Ether | | | 2,00 | 2,00 |
| Cyclomethicon | | | | |
| Dimethicon | 1,00 | | 2,00 | 2,00 |
| Isohexadecan | | 3,00 | | |
| Methylpropandiol | | 4,00 | | |
| Glycerin | 5,00 | | 6,00 | 6,00 |
| NeoHeliopan® AP | | 2,00 | | |
| Phenylbenzimidazol Sulfonsäure | 1,00 | 4,00 | 1,00 | 1,00 |
| Ethylhexylmethoxycinnamat | 5,00 | | 4,00 | 4,00 |
| Diethylhexylbutamidotriazon | 1,00 | | | |
| Butyl Methoxydibenzoylmethan | 2,50 | | 2,00 | 2,00 |
| Bis-Ethylhexyloxyphenol Methoxyphenyltriazin | 2,00 | | | |
| Vitamin E Acetat | 0,20 | | 0,30 | 0,30 |
| BHT | | 0,05 | | |
| Na2H2EDTA | | | 0,40 | 0,40 |
| Parfüm, Konservierungsmittel | q.s. | q.s. | q.s. | q.s. |
| Farbstoffe, usw. | q.s. | q.s. | q.s. | q.s. |
| Natriumhydroxid | q.s. | q.s. | q.s. | q.s. |
| Kaliumhydroxid | | | | q.s. |
| Wasser | ad 100,0 | ad 100,0 | ad 100,0 | ad 100,0 |

Zur Herstellung des Schaums werden 80- 97 Vol.-% der Emulsion I mit 3-20 Vol.-% eines geeigneten Gases (z.B. Propan/ Butan, Druckluft, Stickstoff) aufgeschäumt.

**3. Dünnflüssige bis sprühbare W/O- Emulsionen**

| | **1** | **2** | **3** | **4** | **5** |
|---|---|---|---|---|---|
| Cetyl Dimethicon Copolyol | 4,00 | | | 2,50 | 3,00 |
| Polyglyceryl-2 Dipolyhydroxystearat | | | 3,00 | | 1,00 |
| Isostearyl Diglyceryl Succinat | | | 0,75 | | 0,30 |
| Lauryl Methicon Copolyol | | | | 2,00 | |
| Polysorbat-65 | | | 2,00 | | 1,50 |
| PEG-100 Stearat | | | | 1,20 | 0,70 |
| Cetearyl Sulfat | | | 0,25 | | 1,00 |
| Dimethicon | | 4,00 | | | 2,00 |
| Cyclomethicon | 12,00 | 20,00 | | 30,00 | 15,00 |
| UVASorb® K2A | | | | 0,50 | |
| Uvinul® A Plus | | 2,00 | 0,50 | 4,00 | 0,25 |
| Bis-Ethylhexyloxyphenol Methoxyphenyl Triazin | 1,00 | | | 0,50 | |
| Dinatrium Phenyl Dibenzimidazol Tetrasulfonat | | 1,50 | | | 2,00 |
| Drometrizol Trisiloxan | | | | 1,00 | |
| 4-Methylbenzylidencampher | 4,00 | | | | |
| Methylen Bis-Benztriazolyl Tetramethylbutylphenol | | | | | 3,00 |
| Ethylhexyl Methoxycinnamat | 3,00 | 4,00 | | | 10,00 |
| Ethylheyl Salicylat | | | 5,00 | | 3,50 |
| Octocrylen | | 5,00 | | 4,00 | |
| Diethylhexyl Butamido Triazon | | 1,00 | | | 6,50 |
| Ethylhexyl Triazon | 3,00 | | | | 4,00 |
| Titandioxid MT-100 Z | | 0,50 | 1,00 | 1,50 | 0,50 |
| Zinkoxid Z-Cote | 2,00 | | | | 4,00 |
| Dicaprylyl Carbonat | 5,00 | | 15,00 | | 4,00 |
| C12-15 Alkyl Benzoat | 7,00 | | 2,00 | | |
| Mineral Öl | 3,00 | | | | 2,00 |
| hydriertes Rizinusöl Dimerdilinoleat (CAS 646054-62-8) | 2,00 | 5,00 | 8,00 | 0,50 | 1,00 |
| Kokosfettsäureglycerid | | 2,00 | | 5,00 | |
| PVP Hexadecen Copolymer | | 0,75 | | | 0,40 |
| Glycerin | 5,00 | 12,50 | | 5,00 | 15,50 |
| Sorbitol | 5,00 | | 10,00 | | |
| EDTA | | 0,15 | 0,03 | | 0,15 |
| Glycin Soja | 0,75 | | | 1,50 | |
| Magnesiumsulfat | 0,75 | 1,00 | | 0,45 | 1,00 |
| DMDM Hydantoin | | 0,05 | | | 0,10 |
| Phenoxyethanol | 1,00 | 0,75 | 0,50 | | 1,00 |
| Alkohol | 2,00 | | | 5,00 | 1,00 |
| Farbstoff, öllöslich | 0,02 | | | | |
| Parfüm | 0,30 | 0,45 | 0,35 | | 0,15 |
| Wassser | ad. 100 | ad. 100 | ad. 100 | ad. 100 | ad. 100 |

**4. W/O Emulsionen (Cremes & Lotions)**

| | **1** | **2** | **3** | **4** | **5** | **6** |
|---|---|---|---|---|---|---|
| Cetyldimethicon Copolyol | | | | | 4,00 | |
| Polyglyceryl-2-dipolyhydroxystearat | 5,00 | 4,50 | | | | 4,50 |
| Polyglyceryl-3-Diisostearat | 1,75 | 1,50 | | | | |
| PEG-30-dipolyhydroxystearat | | | 3,00 | 5,00 | | |
| Bis-Ethylhexyloxyphenol Methoxyphenyl Triazin | | | 3,00 | 1,50 | | |
| Butyl Methoxydibenzoylmethan | | | 4,00 | | | |
| UVASorb® K2A | | | | 2,00 | 2,50 | |
| Uvinul ®A Plus | 3,00 | 1,00 | | 3,00 | 0,25 | 2,50 |
| Phenylbenzmidazol Sulfonsäure | | 4,00 | | | 2,00 | 0,50 |
| Ethylhexyl Methoxycinnamat | | 8,00 | | | 5,00 | 4,00 |
| Diethylhexyl Butamido Triazon | 3,00 | 1,00 | | 1,00 | | 3,00 |
| Ethylhexyl Triazon | | | 4,00 | 2,00 | 4,00 | |
| Octocrylen | 7,00 | | | | | 2,50 |
| Titandioxid Uvinul® T 805 | 2,00 | 1,00 | 5,00 | | | |
| Titandioxid MT-100 TV | | | | | | 2,00 |
| Zinkoxid Z-Cote® HP1 | 2,50 | | | 3,00 | | |
| Corapan TQ® | | | 6,00 | | | |
| Mineralöl | | | 6,00 | 5,00 | | |
| Kokosfettsäureglycerid | 4,00 | 6,50 | | 5,00 | | |
| C12-15 Alkyl Benzoat | | | 2,00 | | 9,00 | |
| hydriertes Rizinusöl Dimerdilinoleat (CAS 646054-62-8) | 4,00 | 1,00 | 7,00 | 8,00 | 1,00 | 8,00 |
| Dicaprylylether | 10,00 | | | | | 7,00 |
| Butylenglycol Dicaprylat/Dicaprat | | | 9,00 | 7,00 | 8,00 | 4,00 |
| Cyclomethicon | 2,00 | | | | | 2,00 |
| PVP Eicosene Copolymer | 0,50 | | | | 1,50 | 1,00 |
| Trinatrium EDTA | 1,00 | | 1,00 | 0,50 | 0,35 | |
| Ethylhexyloxyglycerin | | 0,30 | | | | 0,50 |
| Methylpropandiol | | | | | | 7,50 |
| Glycerin | 5,00 | 7,50 | 6,00 | 8,00 | 7,50 | 2,50 |
| Butylenglykol | | 2,50 | | | | |
| MgSO₄ | 1,00 | 0,50 | 0,30 | 0,30 | 0,50 | |
| Milchsäure & Natriumsalz der Milchsäure | 1,00 | 0,50 | | | | 0,85 |
| Vitamin E | 0,50 | | 0,50 | 1,00 | | 1,00 |
| DMDM Hydantoin | | 0,60 | | | 0,20 | |
| Methylparaben | 0,50 | | | | 0,15 | |
| Phenoxyethanol | 0,50 | 0,40 | 0,50 | 0,60 | 1,00 | 0,60 |
| Dihydroxyaceton | | | | | 5,50 | |
| Alcohol | 3,00 | | 2,00 | 3,00 | | 1,00 |
| Parfüm | 0,20 | | 0,20 | 0,20 | | 0,20 |
| Wasser | ad 100 | ad 100 | ad 100 | ad 100 | ad 100 | ad 100 |

**5. Hydrodispersionen (zur Verwendung als Lotion oder Spray)**

| | **1** | **2** | **3** | **4** | **5** | **6** |
|---|---|---|---|---|---|---|
| Glyceryl Stearat Citrat | | 0,40 | | | | |
| Cetyl Alkohol | | | | | 2,00 | |
| Natrium Carbomer | | | | | 0,30 | |
| Acrylates/C10-30 Alkyl Acrylate Crosspolymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Crosspolymer | | | | 5,00 | | 3,00 |
| UVASorb® K2A | | | | | 3,50 | |
| Uvinul®A Plus | 0,25 | | | 0,50 | 2,00 | 1,50 |
| Butyl Methoxydibenzoylmethan | | | 3,50 | | | |
| Bis-Ethylhexyloxyphenol Methoxypheny Triazin | | 2,00 | | 0,25 | | |
| Terephthaliden Dicampher Sulfonsäure | | | | | | 0,50 |
| Dinatrium Phenyl Dibenzimidazol Tetrasulfonat | 0,75 | | | | | 1,00 |
| Phenylbenzimidazol Sulfonsäure | | | 2,00 | | | |
| Ethylhexyl Methoxycinnamat | | | 7,00 | | 5,00 | 8,00 |
| Butyl Methoxydibenzoylmethan | | 3,50 | | | | |
| Diethylhexyl Butamido Triazon | | | 2,00 | 2,00 | | |
| Ethylhexyl Triazon | 4,00 | 3,00 | | | 4,00 | |
| Octocrylen | | | | 10,00 | | 2,50 |
| Titandioxid MT-100 Z | 0,50 | 2,00 | 1,00 | 2,00 | 3,00 | 1,00 |
| C12-15 Alkyl Benzoat | 2,00 | | 2,50 | | | |
| hydriertes Rizinusöl Dimerdilinoleat (CAS 646054-62- 8) | 1,00 | 0,50 | 0,20 | 1,50 | 2,50 | 5,00 |
| C18-36 Triglycerid Fettsäure | | | 1,00 | | | |
| Butylenglycol Dicaprylat/Dicaprat | | | | | 6,00 | |
| Dicaprylyl Carbonat | | 3,00 | | | | |
| Dicaprylylether | | 2,00 | | | | |
| Cyclomethicon | | | | 1,50 | | |
| Lanolin | | | | | 0,35 | |
| PVP Hexadecen Copolymer | 0,50 | | 0,50 | | 0,50 | 1,00 |
| Ethylhexyloxyglycerin | | 0,75 | | 1,00 | | 0,50 |
| Glycerin | 10,00 | 5,00 | 5,00 | | 5,00 | 15,00 |
| Butylenglycol | | 7,00 | | | | |
| Glycin Soja | | | | 1,00 | | |
| Vitamin E Acetat | 0,50 | 0,25 | 0,50 | 0,25 | 0,75 | 1,00 |
| α - Glycosylrutin | | | | | 0,25 | |
| Trinatrium EDTA | | 1,00 | 1,00 | 0,10 | 0,20 | |
| lodopropinylbutylcarbamat | 0,20 | 0,10 | | | | 0,15 |
| Methylparaben | 0,50 | | 0,20 | | 0,15 | |
| Phenoxyethanol | 0,50 | 0,40 | 0,40 | | 1,00 | 0,60 |
| Ethanol | 3,00 | 10,00 | 4,00 | 3,50 | | 1,00 |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |
| Neutralisationsmittel (Natriumhydroxid, Kaliumhydroxid) | qs | qs | qs | qs | qs | qs |
| pH-Wert | 4,5-5,5 | 5,0-7,0 | 5,0-7,0 | 5,0-7,0 | 4,0-6,0 | 5,0-7,5 |

**5. Gelcremes**

| | **1** | **2** | **3** | **4** | **5** |
|---|---|---|---|---|---|
| Carbomer | 0,125 | 0,125 | 0,125 | 0,125 | 0,125 |
| Shea Butter | | | | 1,00 | |
| Mineralöl | | | | | 6,00 |
| Octyldodecanol | | | | 1,50 | |
| Caprylsäure/Caprinsäuretriglycerid | | | 4,00 | | |
| Dicaprylyl Carbonat | | 9,00 | | 1,00 | 3,00 |
| Phenethyl Benzoat | 1,00 | | 3,00 | | 2,50 |
| hydriertes Rizinusöl Dimerdilinoleat 0,50 (CAS 646054-62-8) | 0,50 | 1,00 | 2,50 | 6,00 | 0,75 |
| Dimethicon | | | | 0,50 | |
| Cyclomethicon | 9,00 | 2,00 | 3,00 | 2,00 | 1,00 |
| Diazolidinylharnstoff | 0,20 | 0,20 | 0,20 | 0,20 | 0,20 |
| Phenoxyethanol + Ethyl-, Methyl-, Propyl- Butyl-, Isobutylparaben | 0,50 | 0,50 | 0,50 | 0,50 | 0,50 |
| Parfum | | | | 0,25 | 0,25 |
| Glycerylstearatcitrat | 1,00 | 1,00 | 1,00 | 1,00 | 1,00 |
| Hydrierte Kokosfettsäureglycerid | 1,00 | 1,00 | 1,00 | 1,00 | 1,00 |
| Ammonium Acryloyldimethyltaurat/VP Copolymer | | | | | 0,125 |
| Hydroxyethylcellulose | 0,375 | 0,375 | 0,375 | 0,375 | 0,375 |
| Menthol | 0,10 | 0,50 | 1,00 | 0,10 | 0,10 |
| Wasser + Alcohol Denat | | | | 3,00 | |
| Wasser + Blue 1 | | 0,200 | 0,60 | 0,40 | |
| Wasser + Glycerin | 10,00 | 10,00 | 10,00 | 10,00 | 10,00 |
| Xanthangummi | 0,125 | 0,125 | 0,125 | 0,125 | |
| Wasser | ad 100,0 | ad 100,0 | ad 100,0 | ad 100,0 | ad 100,0 |
| Neutralisationsmittel (Natriumhydroxid, Kaliumhydroxid) | qs | qs | qs | qs | qs |
| pH-Wert | 4,5-5,5 | 6,5-8,5 | 5,0-7,0 | 4,0-6,0 | 5,0-7,5 |

**6. Ölgele**

| | **1** | **2** | **3** | **4** | **5** |
|---|---|---|---|---|---|
| Octyldodecanol | 9,00 | 9,00 | | | |
| Caprylsäure/Caprinsäuretriglycerid | 1,00 | | 1,00 | | |
| C12-15- Alkyl Benzoate | | | | 5,00 | 4,00 |
| Butylenglycol Dicaprylat/Dicaprat | | 9,00 | | | 3,00 |
| hydriertes Rizinusöl Dimerdilinoleat (CAS 646054-62- 8) | 2,00 | 1,00 | 1,00 | 0,25 | 5,00 |
| Dicaprylylether | 9,00 | | | 4,00 | |
| Dicaprylylcarbonat | | 7,00 | | | |
| Ethyl Galaktomannan (N-Hance® AG 200) | 3,50 | | | | 4,00 |
| C20-40 Fettsäuren + Polyethylen (Performacid®350) | | | | 3,60 | |
| Hydroxyoctacosanyl Hydroxystearat | 2.00 | | | | |
| Disteardimonium Hectorit | 1,00 | | | | 1,00 |
| Cetyl Dimethicon | 0,50 | | 4,50 | | |
| Cyclomethicon | | | 15,00 | | 5,00 |
| UVASorb® K2A | | | | | 1,00 |
| Uvinul®A Plus | 1,00 | 3,50 | 2,75 | 2,00 | 0,50 |
| Ethylhexylmethoxycinnamat | 6,00 | | | 10,00 | 3,0 |
| Octocrylen | 3,50 | | 7,50 | 10,00 | |
| Ethylhexylsalicylat | | 3,50 | | | 4,00 |
| Ethylhexyl Triazon | | | 2,00 | | |
| Diethylhexyl Butamido Triazon | | 0,50 | | 3,00 | 4,0 |
| Polysaccharid-N-alkylurethanen, Inulincarbamat | 1,50 | 4,50 | 5,50 | 1,00 | 3,00 |
| Phenoxyethanol | 0,50 | | | | |
| Parfüm, Farbstoff | q.s. | q.s. | q.s. | q.s. | q.s. |
| Mineralöl | ad. 100 | ad. 100 | ad. 100 | | |
| Reisöl | | | | ad. 100 | ad. 100 |

**7. Feststoffstablisierte Emulsionen**

| | **1** | **2** | **3** | **4** | **5** |
|---|---|---|---|---|---|
| Mineralöl | | | 16,00 | 16,00 | |
| Octyldodecanol | 9,00 | 9,00 | 5,00 | | |
| Caprylic/Capric Triglycerid | 9,00 | 4,00 | 6,00 | | |
| C12-15- Alkyl Benzoat | | | | | 3,00 |
| Butylene Glykol Dicaprylat/Dicaprat | | | | | 3,00 |
| hydriertes Rizinusöl Dimerdilinoleat (CAS 646054-62-8) | 2,00 | 1,00 | 1,00 | 2,50 | 0.50 |
| Dicaprylyl Ether | 9,00 | | | 4,00 | |
| Dicaprylyl Carbonat | | 9,00 | | | |
| Hydroxyoctacosanyl Hydroxystearat | 2,00 | 2,00 | 2,00 | 2,00 | 1,50 |
| Disteardimonium Hectorit | 1,00 | 0,750 | 0,50 | 0,50 | 0,25 |
| Cera Microcristallina + Paraffinöl | | | 2,50 | | 5,00 |
| Hydroxypropyl Methylcellulose | 0,15 | | | | 0,05 |
| Dimethicon | | | 4,50 | | |
| UVASorb® K2A | 2,00 | 5,00 | 3,00 | 1,50 | 1,00 |
| Bis-Ethylhexyloxyphenol Methoxypheny Triazin | 1,00 | 3,00 | 0,75 | 1,00 | 1,00 |
| Terephthaliden Dicampher Sulfonsäure | | 2,00 | | | 0,50 |
| Phenylbenzmidazol Sulfonsäure | 2,00 | 0,50 | 1,00 | | |
| Uvinul® A Plus | | | 2,75 | | 0,50 |
| Ethylhexylmethoxycinnamat | 6,00 | | | | 3,0 |
| Octocrylen | 3,50 | | 7,50 | | |
| Ethylhexyl Salicylat | | 3,50 | | | 4,00 |
| Diethylhexyl Butamido Triazon | | | | | 4,0 |
| Titandioxid Eusolex® T-2000 | | 2,00 | 4,00 | 2,00 | 4,00 |
| Titandioxid T 805® | | | | | 3,00 |
| Silica Dimethyl Silylat | | | 1,00 | | |
| Bornitrid | 2,00 | | | 3,00 | |
| Tapioca Stärke | | | | 1,00 | |
| Natriumchlorid | 1,00 | 1,00 | 1,00 | 1,00 | |
| Glycerin | 5,0 | 10,0 | | 6,00 | 10,0 |
| Trinatrium EDTA | | 1,00 | | 1,00 | |
| Methylparaben | 0,21 | | | | 0,20 |
| Propylparaben | 0,07 | | | | |
| Phenoxyethanol | 0,50 | | 0,40 | 0,40 | 0,50 |
| Hexamidin Diisethionat | | | | | 0,08 |
| Diazolidinyl Harnstoff | | | 0,28 | 0,28 | |
| Alkohol | | 5,00 | | 2,50 | |
| Parfüm | 0,45 | 0,20 | | | 0,45 |
| Wasser | ad 100 | ad 100 | ad 100 | ad 100 | ad 100 |

### 8. Gele:

### Beispiel 1 (Eye Shadow Gel):

| | Gew.-% |
|---|---|
| PEG-8 (Polyethylenglycol 400) | 2,00 |
| Ethanol | 5,00 |
| Aristoflex AVC | 1,50 |
| hydriertes Rizinusöl Dimerdilinoleat (CAS 646054-62-8) | 1,00 |
| Glycerin | 2,00 |
| Panthenol | 0,50 |
| Tocopherolacetat | 0,50 |
| Timiron Splendid blue® (Merck KgaA) | 4,50 |
| Chromoxid grün | 1,00 |
| Parfüm, Konservierungsmittel, NaOH, Komplexbildner, Farbstoffe, Antioxidantien etc. | q.s. |
| Wasser | ad 100,00 |

### Beispiel 2 (Hiqhliqhter Gel):

| | Gew. % |
|---|---|
| Carbomer | 1,50 |
| Glycerin | 2,50 |
| 1,3 Butylenglycol | 1,50 |
| hydriertes Rizinusöl Dimerdilinoleat (CAS 646054-62-8) | 1,50 |
| Glitzerpigmente (z.B. Helicone HC Scarabeus, Wacker) | 1,00 |
| EDTA | 0,20 |
| Dimethicone | 1,50 |
| Parfüm, Konservierungsmittel, NaOH, Farbstoffe, Antioxidantien, etc. | q.s. |
| Wasser | ad 100,00 |

### Beispiel 3 (Eye Liner Gel):

| | Gew. % |
|---|---|
| Perlglanzpigmente | 10,00 |
| Eisenoxid | 3,00 |
| Silica | 2,00 |
| Aristoflex AVC | 1,00 |
| Hyroxyproylethyl Cellulose | 0,35 |
| Citric Acid | q.s. |
| Glycerin | 5,00 |
| hydriertes Rizinusöl Dimerdilinoleat (CAS 646054-62-8) | 0,50 |
| PVP / VA Copolymer | 2,00 |
| Parfüm, Konservierungsmittel, Farbstoffe, NaOH, Komplexbildner, Antioxidantien, etc. | q.s. |
| Wasser | ad 100,00 |

### Make-up

### Beispiel 1 (Emulsions-Make-up):

| | Gew. % |
|---|---|
| PEG-30 Stearat | 2,00 |
| Glycerinmonostearat | 1,00 |
| Stearinsäure | 1,00 |
| Cyclomethicon | 7,00 |
| Octyldodecanol | 4,00 |
| hydriertes Rizinusöl Dimerdilinoleat (CAS 646054-62-8) | 2,00 |
| Isopropyl Lanolat | 4,00 |
| Squalan | 2,00 |
| Octyl Methoxycinnamat | 2,00 |
| Butyl Methoxydibenzoylmethan | 1,00 |
| Xanthan Gum | 0,20 |
| Glycerin | 5,00 |
| Butylenglkcol | 2,00 |
| Vitamin E Acetat | 1,00 |
| Magnesiumsilikat | 1,00 |
| Glimmer | 1,00 |
| Eisenoxid | 1,00 |
| Titandioxid | 2,50 |
| Talkum | 5,00 |
| EDTA | 0,50 |
| Parfüm, Konservierungsmittel, NaOH, Antioxidantien etc. | q.s. |
| Wasser | ad 100,00 |

### Beispiel 2 (Emulsions-Make-up):

| | Gew. % |
|---|---|
| Cyclomethicon | 18,00 |
| Phenyltrimethicon | 3,00 |
| hydriertes Rizinusöl Dimerdilinoleat (CAS 646054-62-8) | 0,75 |
| Cetyl PEG/PPG - 10/1 Dimethicon (z.B. Abil EM 90) | 4,00 |
| Paraffinöl | 3,00 |
| Eisenoxid | 2,30 |
| Titandioxid | 4,50 |
| Talkum | 2,00 |
| Natrium Chlorid | 2,00 |
| Quaternium-18 Hectorit | 0,30 |
| Propylen Carbonat | 0,08 |
| Parfüm, Konservierungsmittel, NaOH, Antioxidantien etc. | q.s. |
| Wasser | ad 100,00 |

## Patentansprüche

1. Kosmetische Zubereitung enthaltend
a) ein oder mehrere UV-Lichtschutzfilter sowie
b) hydriertes Rizinusöl Dimerdilinoleat (CAS 646054-62-8),
**dadurch gekennzeichnet, dass** das Gewichtsverhältnis der UV-Lichtschutzfilter a) zu hydriertem Rizinusöl Dimerdilinoleat b) von 15:1 bis 1:1 beträgt.

2. Kosmetische Zubereitung nach Anspruch 1 enthaltend
a) 4-Methoxyzimtsäure(2-ethylhexyl)ester (CAS 5466-77-3) und/oder 4-(tert.-Butyl)-4'-methoxydibenzoylmethan (CAS 70356-09-1) in einer Gesamtkonzentration von 2 bis 15 Gewichts-%, bezogen auf das Gesamtgewicht der Zubereitung sowie
b) hydriertes Rizinusöl Dimerdilinoleat (CAS 646054-62-8).

3. Kosmetische Zubereitung nach Anspruch 1 enthaltend
a) 2,4-Bis-{[4-(2-ethyl-hexyloxy)-2-hydroxy]-phenyl}-6-(4-methoxyphenyl)-1,3,5-triazin und/oder 4,4',4"-(1,3,5-Triazin-2,4,6-triyltriimino)-tris-benzoesäure-tris(2-ethylhexylester) in einer Gesamtkonzentration von 0,1 bis 10 Gewichts-%, bezogen auf das Gesamtgewicht der Zubereitung sowie
b) hydriertes Rizinusöl Dimerdilinoleat (CAS 646054-62-8).

4. Kosmetische Zubereitung nach einem der Ansprüche 1 bis 3, **dadurch gekennzeichnet, dass** die Zubereitung hydriertes Rizinusöl Dimerdilinoleat (CAS 646054-62-8) in einer Gesamtkonzentration von 0,1 bis 20 Gewichts-%, bezogen auf das Gesamtgewicht der Zubereitung, enthält.

5. Kosmetische Zubereitung nach einem der Ansprüche 2, 3 und 4, **dadurch gekennzeichnet, dass** sie weitere UV-Lichtschutzfilter gewählt aus der Gruppe der UV-Lichtschutzfilter 2,4-Bis-{[4-(2-ethyl-hexyloxy)-2-hydroxyl-phenyl}-6-(4-methoxyphenyl)-1,3,5-triazin; Dioctylbutylamidotriazon; 2,4-bis-[5-1(dimethylpropyl)benzoxazol-2-yl-(4-phenyl)-imino]-6-(2-ethylhexyl)-imino-1,3,5-triazin; 4,4',4"-(1,3,5-Triazin-2,4,6-triyltriimino)-tris-benzoësäure-tris(2-ethylhexylester); 2,4,6-Tribiphenyl-4-yl-1,3,5-triazin; Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; Ethylhexylsalicylat; Terephthalidendicamphersulfonsäure; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl}-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(Dimethylamino)-benzoesäure(2-ethylhexyl)ester; 4-(Dimethylamino)benzoesäure-amylester; 4-Methoxybenzalmalonsäuredi(2-ethylhexyl)ester; Dioctylbutylamidotriazon; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; 2-Ethylhexyl-2-cyano-3,3-diphenylacrylat; 3-(4-(2,2-bis Ethoxycarbonylvinyl)-phenoxy)propenyl)-methoxysiloxan / Dimethylsiloxan - Copolymer; Dimethicodiethylbenzalmalonat; Titandioxid; Zinkoxid enthält.

6. Kosmetische Zubereitung nach einem der Ansprüche 2, 3 und 4, **dadurch gekennzeichnet, dass** sie weitere UV-Lichtschutzfilter gewählt aus der Gruppe der UV-Lichtschutzflter Dioctylbutylamidotriazon; 2,4-bis-[5-1(dimethylpropyl)benzoxazol-2-yl-(4-phenyl)-imino]-6-(2-ethylhexyl)-imino-1,3,5-triazin; 2,4,6-Tribiphenyl-4-yl-1,3,5-triazin; Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzotsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(Dimethylamino)-benzoesäure(2-ethylhexyl)ester; 4-(Dimethylamino)benzoesäure-amylester; 4-Methoxybenzalmalonsäuredi(2-ethylhexyl)ester; Dioctylbutylamidotriazon; 4-Methoxyzimtsäure(2-ethylhexyl)ester; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester 4-(tert.-Butyl)-4'-methoxydibenzoylmethan; Ethylhexylsalicylat; Terephthalidendicamphersulfonsäure; Homomenthylsalicylat; 2-Ethylhexyl-2-hydroxybenzoat; 2-Ethylhexyl-2-cyano-3,3-diphenylacrylat; 3-(4-(2,2-bis Ethoxycarbonylvinyl)-phenoxy)propenyl)-methoxysiloxan / Dimethylsiloxan - Copolymer; Dimethicodiethylbenzalmalonat; Titandioxid; Zinkoxid enthält.

7. Kosmetische Zubereitung nach einem der vorhergehenden Ansprüche, **dadurch gekennzeichnet, dass** die Zubereitung Alpha-Hydroxysäuren und/oder deren Salze enthält.

8. Kosmetische Zubereitung nach einem der vorhergehenden Ansprüche, **dadurch gekennzeichnet, dass** die Zubereitung Tocopherylacetat enthält.

9. Kosmetische Zubereitung nach einem der vorhergehenden Ansprüche, **dadurch gekennzeichnet, dass** die Zubereitung in Form einer Emulsion vorliegt.

## Claims

1. Cosmetic preparation comprising
a) one or more UV photoprotective filters and
b) hydrogenated castor oil dimer dilinoleate (CAS 646054-62-8),
**characterized in that** the weight ratio of the UV photoprotective filters a) to hydrogenated castor oil dimer dilinoleate b) is from 15:1 to 1:1.

2. Cosmetic preparation according to Claim 1, comprising
a) 2-ethylhexyl 4-methoxycinnamate (CAS 5466-77-3) and/or 4-(tert-butyl)-4'-methoxydibenzoylmethane (CAS 70356-09-1) in a total concentration of from 2 to 15% by weight, based on the total weight of the preparation, and
b) hydrogenated castor oil dimer dilinoleate (CAS 646054-62-8).

3. Cosmetic preparation according to Claim 1, comprising
a) 2,4-bis{[4-(2-ethylhexyloxy)-2-hydroxy]phenyl}-6-(4-methoxyphenyl)-1,3,5-triazine and/or tris(2-ethylhexyl) 4,4',4"-(1,3,5-triazine-2,4,6-triyltriimino)trisbenzoate in a total concentration of from 0.1 to 10% by weight, based on the total weight of the preparation, and
b) hydrogenated castor oil dimer dilinoleate (CAS 646054-62-8).

4. Cosmetic preparation according to one of Claims 1 to 3, **characterized in that** the preparation comprises hydrogenated castor oil dimer dilinoleate (CAS 646054-62-8) in a total concentration of from 0.1 to 20% by weight, based on the total weight of the preparation.

5. Cosmetic preparation according to one of Claims 2, 3 and 4, **characterized in that** it comprises further UV photoprotective filters selected from the group of the UV photoprotective filters 2,4-bis{[4-(2-ethylhexyloxy)-2-hydroxy]phenyl}-6-(4-methoxyphenyl)-1,3,5-triazine; dioctylbutylamidotriazone; 2,4-bis[5-1(dimethylpropyl)benzoxazol-2-yl-(4-phenyl)imino]-6-(2-ethylhexyl)imino-1,3,5-triazine; tris(2-ethylhexyl) 4,4',4"-(1,3,5-triazine-2,4,6-triyltiimino)trisbenzoate; 2,4,6-tribiphenyl-4-yl-1,3,5-triazine; phenylene-1,4-bis(2-benzimidazyl)-3,3'-5,5'-tetrasulphonic acid salts; 2-phenylbenzimidazole-5-sulphonic acid salts: 1,4-di(2-oxo-10-sulpho-3-bornylidenemethyl)benzene and salts thereof; 4-(2-oxo-3-bornylidenemethyl)benzenesulphonic acid sales; ethylhexyl salicylate; terephthalidenedicamphorsulphonic acid; 2-methyl-5-(2-oxo-3-bornylidenemethyl)-sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-(1,3,3,3-tetramethyl-1-[(trimethylsilyl)oxy]disiloxanyl]propyl]phenol; 3-(4-methylbenzylidene)camphor; 3-benzylidenecamphor; 2-ethylhexyl 4-(dimethylamino)benzoate; amyl 4-(dimethylamino)benzoate; di(2-athylhexyl) 4-methoxybenzalmalonate; dioctylbutylamidotriazone; isopentyl 4-methoxycinnamate; 2-hydroxy-4-methoxybenzophenone, 2-hydroxy-4-methoxy-4'-methylbenzophenone; 2,2'-dihydroxy-4-methoxybenzophonone; hexyl 2-(4'-diethylamino-2'-hydroxybenzoyl)benzoate; homomenthyl salicylate; 2-ethylhexyl 2-hydroxybenzoate; 2-ethylhexyl 2-cyano-3,3-diphenylacrylate; 3-(4-(2,2-bis-ethoxycarbonylvinyl)phenoxy)propenyl)methoxysiloxaneldimethylsiloxane copolymer; dimethicodiethyl benzalmalonate; titanium dioxide; zinc oxide.

6. Cosmetic preparation according to one of Claims 2, 3 and 4, **characterized in that** it comprises further UV photoprotective filters selected from the group of the UV photoprotective filters dioctylbutylamidotriazone; 2.4-bis[5-1(dimethylpropyl)benzoxazol-2-yl-(4-phenyl)imino]-6-(2-ethylhexyl)imino-1,3,5-triazine; 2,4,6-tribiphenyl-4-yl-1,3,5-triazine; phenylene-1,4-bis(2-banzimidazyl)-3,3'-5,5'-tetrasulphonic acid salts; 2-phenylbenzimidazole-5-sulphonic acid salts; 1,4-di(2-oxa-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenoll; 3-(4-methylbenzylidene)camphor; 3-benzylidenecamphor; 2-ethylhexyl 4-(dimethylamino)benzoate; amyl 4-(dimethylamino)benzoate; di(2-ethylhexyl) 4-methoxybenzalmalonate; dioctylbutylamidotriazone; 2-ethylhexyl 4-methoxycinnamate; isopentyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate; 4-(tert-butyl)-4'-methoxydibenzoylmethane; ethylhexyl salicylate; terephthalidenedicamphorsulphonic acid; homomenthyl salicylate; 2-ethylhexyl 2-hydroxybenzoate; 2-ethylhexyl 2-cyano-3,3-diphenylacrylate; 3-(4-(2,2-bis-ethoxycarbonylvinyl)phenoxy)propenyl)methoxysiloxaneldimethylsioxane copolymer; dimethicodiethyl benzalmalonate; titanium dioxide; zinc oxide.

7. Cosmetic preparation according to one of the preceding claims, **characterized in that** the preparation comprises alpha-hydroxy acids and/or salts thereof.

8. Cosmetic preparation according to one of the preceding claims, **characterized in that** the preparation comprises tocopheryl acetate.

9. Cosmetic preparation according to to one of the preceding claims, **characterized in that** the preparation is in the form of an emulsion.

## Revendications

1. Préparation cosmétique contenant
a) un ou plusieurs filtres de protection contre la lumière UV ainsi que
b) de l'ester d'huile de ricin hydrogénée et d'acide dilinoléique dimère (CAS 646054-62-8),
**caractérisée en ce que** le rapport pondéral du filtre de protection contre la lumière UV a) à l'ester d'huile de ricin hydrogénée et d'acide dilinoléique dimére b) est de 15:1 à 1:1.

2. Préparation cosmétique selon la revendication 1, contenant
a) de l'ester 2-éthylhexylique de l'acide 4-méthoxycinnamique (CAS 5466-77-3) et/ou du 4-(tert-butyl)-4'-méthoxydibonzoylméthane (CAS 70356-09-1) en une concentration totale de 2 à 15% en poids par rapport au poids total de la préparation, ainsi que
b) de l'ester d'huile de ricin hydrogénée et d'acide dilinoléique dimère (CAS 646054-62-8).

3. Préparation cosmétique selon la revendication 1, contenant
a) de la 2,4-bis-{[4-(2-éthylhexyloxy)-2-hydroxy]phényl}-6-(4-méthoxyphényl)-1,3,5-triazine et/ou de l'ester tris(2-éthylhexylique) de l'acide 4,4',4"-(1,3,5-triazine-2,4,6-triyltriimino)-trisbenzoïque en une concentration totale de 0,1 à 10% en poids par rapport au poids total de la préparation, ainsi que
b) de l'ester d'huile de ricin hydrogénée et d'acide dilinoléique dimère (CAS 646054-62-8).

4. Préparation cosmétique selon l'une quelconque des revendications 1 à 3, **caractérisée en ce que** la préparation contient de l'ester d'huile de ricin hydrogénée et d'acide dilinoléique dimère (CAS 646054-62-8) en une concentration totale de 0,1 à 20% en poids, par rapport au poids total de la préparation.

5. Préparation cosmétique selon l'une quelconque des revendications 2, 3 et 4, **caractérisée en ce qu'**elle contient d'autres filtres de protection contre la lumière UV, choisis dans le groupe des filtres de protection contre la lumière UV 2,4-bis-([4-(2-éthylhexyloxy)-2-hydroxy]-phényl)-6-(4-methoxyphenyl)-1,3,5-triazine ; dioctylbutylamidotriazone ; 2,4-bis-[5-1(diméthylpropyl)benzoxazol-2-yl-(4-phényl)-imino]-6-(2-éthylhexyl)-imino-1,3,5-triazine ; ester tris(2-éthylhexylique) de l'acide 4,4',4"-(1,3,5-triazine-2.4,6-triyltriimino)-trisbenzoïque ; 2,4,6-tribiphériyl-4-yl-1,3,5-triazine ; sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzénesulfonique ; salicylate d'éthylhexyle ; acide téréphtalidènedicamphresulfonique ; sels de l'acide 2-méthyl-5-(2-oxo-3-bornylidèneméthyl)sulfonique ; 2,2'-méthylénebis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; ester 2-éthylhexylique de l'acide 4-(diméthylamino)-benzoïque ; ester amylique de l'acide 4-(diméthylamino)benzeïque ; ester di(2-éthylhexylique) de l'acide 4-méthoxybenzalmalonique ; dioctylbutylamidotriazone ; ester isopentylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone , ester hexylique de l'acide 2-(4'-diéthylamino-2'-hydoxybenzoyl)-benzoïque ; salicylate d'homomenthyle ; 2-hydroxybenzoate de 2-éthylhexyle ; 2-cyan-3,3-diphénylacrylate de 2-éthylhexyle ; copolymère de 3-(4-(2,2-biséthoxycarbonylvinyl)-phénoxy)propényl)-méthaxysiloxane/diméthylsiloxane ; benzalmalonate de diméthicodiéthyle ; dioxyde de titane ; oxyde de zinc.

6. Préparation cosmétique selon l'une quelconque des revendications 2, 3 et 4, **caractérisée en ce qu'**elle contient d'autres filtres de protection contre la lumière UV, choisis dans le groupe des filtres de protection contre la lumière UV dioctylbutylamidotriazone ; 2,4-bis-[5-1(diméthylpropyl)benzoxazol-2-yl-(4-phényl)-imino]-6-(2-éthylhexyl)-imino-1,3,5-triazine ; 2,4,8-tribiphényl-4-yl-7,3,5-triazine ; sels de l'acide phénylène-1,4-bis-(2-benzimidazyl)-3,3'-5,5'-tétrasulfonique; sels de l'acide 2-phénylbenzimidazole-5-sulfonique ; 1,4-di(2-oxo-10-sulfo-3-bornylidéneméthyl)-benzène et ses sels ; sels de l'acide 4-(2-oxo-3-bornylidéneméthyl)benzènesulfonique ; sels de l'acide 2-méthyl-5-(2-oxo)-3-bornylidèneméthyl)sulfonique ; 2,2'-méthylènebis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phéncl ; 3-(4-méthylbenzylidène)camphre ; 3-benzylidènecamphre ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; dioctylbutylamidotriazone ; ester 2-éthylhexylique de l'acide 4-méthoxycinnamique ; ester isopentylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone , ester hexylique de l'acide 2-(4'-diéthylamino-2'-hydroxybenzoyl)-benzoïque ; 4-(tert-butyl)-4'-méthoxydibenzoylméthane ; salicylate d'éthylhexyle ; acide téréphtalidènedicamphresulfonique ; salicylate d'homomenthyle ; 2-hydroxybenzoate de 2-éthylhexyle ; 2-cyano-3,3-diphénylacrylate de 2-éthylhexyle ; copolymère de 3-(4-(2,2-biséthoxycarbonylvinyl)-phénoxy)propényl)-méthoxysiloxane/diméthylsiloxane ; benzalmalonate de diméthicodièthyle ; dioxyde de titane ; oxyde de zinc.

7. Préparation cosmétique selon l'une quelconque des revendications précédentes, **caractérisée en ce que** la préparation contient des alpha-hydroxyacides et/ou leurs sels.

8. Préparation cosmétique selon l'une quelconque des revendications précédentes, **caractérisée en ce que** la préparation contient de l'acétate de tocophéryle.

9. Préparation cosmétique selon l'une quelconque des revendications précédentes, **caractérisée en ce que** la préparation se trouve sous forme d'une émulsion.
